Europäisches Patentamt

European Patent Office

Office européen des brevets

(19)

(11) Publication number: **0 264 036 B1**

(12) **EUROPEAN PATENT SPECIFICATION**

(45) Date of publication of patent specification: **30.06.93**

(51) Int. Cl.[5]: **G01N 33/52**, G01N 33/571, G01N 33/545, G01N 33/546

(21) Application number: **87114415.0**

(22) Date of filing: **02.10.87**

(54) **Analytical device and method for detecting chlamydia trachomatis and neisseria gonorrhoeae.**

(30) Priority: **16.10.86 US 919396**

(43) Date of publication of application:
**20.04.88 Bulletin 88/16**

(45) Publication of the grant of the patent:
**30.06.93 Bulletin 93/26**

(84) Designated Contracting States:
**AT BE CH DE ES FR GB IT LI NL**

(56) References cited:
**EP-A- 0 119 622       EP-A- 0 173 375
EP-A- 0 200 381       EP-A- 0 217 403
EP-A- 0 269 876       US-A- 4 042 329
US-A- 4 338 094**

(73) Proprietor: **ABBOTT LABORATORIES**

**Abbott Park, Illinois 60064(US)**

(72) Inventor: **Varitek, Vincent Andrew, Jr.**
**33139 North Sunset**
**Wildwood, IL 60030(US)**

(74) Representative: **Modiano, Guido et al**
**c/o Modiano & Associati S.r.l. Via Meravigli, 16**
**I-20123 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.6/3.3.1)

## Description

Background of the Invention

The present invention is generally directed to a device and method for accurately detecting Chlamydia trachomatis and Neisseria gonorrhoeae antigens. The detection of these two types of antigens is complicated by the difficulty in obtaining sufficient quantity of sample to test and by the low concentration of antigen present in such samples. Therefore it is desirable to design a device and method whereby minimal amounts of sample can be accurately tested and minimal amounts of the particular antigen detected.

One device which can be employed to identify antigens by performing a binding assay is TESTPACK™ manufactured by Abbott Laboratories, Abbott Park, Illinois and disclosed in EP-A-0 217 403 which is a prior European application falling within the terms of Article 54(3) EPC.

Generally the device employs an enzyme immunoassay technique and comprises a plurality of substantially spherical, solid particles immobilized within a porous fiber matrix. The particles may have a substance coated on their surface which is capable of reacting with analyte present in a sample. The sample is brought into contact with the porous matrix where analyte present in the sample binds to the microparticle surface where it can be detected.

Other methods have been developed to identify Chlamydia trachomatis and Neisseria gonorrhoeae as disclosed in U.S. Patent 4,497,899 and 4,497,900. In particular these patents disclose absorbing the respective antigens onto a solid phase where they are then treated with antibody, washed, treated with antiglobulin, washed and, finally the amount of antiglobulin bound to the antigen antibody complex determined. Despite these methods, further improvements are necessary to identify small concentrations of antigen or analyze small quantities of sample for Chlamydia trachomatis or Neisseria gonorrhoeae. These problems have been identified and their solutions are the subject matter of the present invention.

Summary of the Invention

In one aspect, the present invention is directed toward a solid-phase assay device useful in a binding assay to determine the presence of Chlamydia trachomatis or Neisseria gonorrhoeae antigen in a sample as defined in the claims. The solid-phase device comprises a substantially planar layer of a material having a porous matrix of fibers and characterized by a plurality of substantially spherical, solid particles comprising polytetrafluoroethylene having an average diameter of from 0.1 to 10 microns. The particles are retained and immobilized within the matrix upon the fibers. The substantially planar layer has a first, sample-contacting surface and a second surface opposed to the first surface, and is disposed in the device such that, when the device is performing the assay, at least a portion of the sample contacting the first surface passes through the substantially planar layer to the second surface. The polytetrafluoroethylene particles serve to bind the antigen which can then be identified by immunoenzyme technique. The device can further employ on-board controls to assure accurate identification and readily recognizable results.

In another aspect, the present invention is directed toward a method for performing a binding assay to determine the presence of Chlamydia trachomatis or Neisseria gonorrhoeae antigen in a test sample. The steps comprise:

a) contacting a porous matrix of fibers having a plurality of substantially spherical, solid particles comprising polytetrafluoroethylene having an average diameter of from 0.1 to 10 $\mu$m, the particles being retained and immobilized within the matrix upon the fibers, whereby antigen in the sample becomes bound to the particles forming an antigen complex on the particles;

b) contacting the antigen complex on the particles with a second substance capable of reaction with the antigen which is labeled with a substance capable of producing a detectable response in the presence of the antigen and an indicator substance, whereby the labeled substance becomes bound to the antigen complex on the particles;

c) removing unbound labeled substance from the material;

d) contacting the complex with the indicator substance; and

e) detecting the response produced as a function of the presence or amount of the antigen in the sample.

In yet another aspect, the subject invention is directed toward a method for detecting the presence of Chlamydia trachomatis or Neisseria gonorrhoeae antigen in a fluid sample. This method comprises the steps of:

a) incubating with a fluid sample a plurality of substantially spherical, solid particles comprising polytetrafluoroethylene having an average diameter of from about 0.1 to about 10 $\mu$m, whereby the

antigen becomes bound to the particles forming an antigen complex on the particles; and

b) contacting a porous, fibrous matrix with the incubated particles, whereby at least a portion of the particles become retained and immobilized within the matrix upon at least a portion of the fibers.

c) detecting the presence of antigen bound to the particles.

In said step c), the bound particle complex is observed by treatment with an enzyme-conjugated antibody or antigen which is treated with an indicator substance such that the complex can be observed either visually or with instrumentation.

The subject invention is characterized by employing microparticles of polytetrafluorethylene to effectively bind the Chlamydia trachomatis or Neisseria gonorrhoeae antigen. Polytetrafluoroethylene has been found to be superior to other microparticles. This is especially valuable because generally the samples of interest are small in quantity or low in concentration of antigen.

## Brief Description of the Drawings

Fig. 1 is a side view in partial cross section of an analytical device suitable for use in the subject invention.

Fig. 2. is a top plan view of the device of Fig. 1.

Figs. 3A, 3B, 3C and 4A, 4B, 4C are top plan views of various embodiments of the device of Fig. 1 and depicting various examples of control areas.

Fig 5. is a perspective view of the device of Fig. 1, showing pre-filter assembly removed from the body of the device.

## Detailed Description of the Invention

The present invention is directed toward an improved method for identifying Chlamydia trachomatis - (Chlamydia) and Neisseria gonorrhoeae (gonorrhoeae) antigen present in a sample. In particular the subject device and method is adapted to detecting the presence of small quantities of Chlaymdia and gonorrhoeae antigens present in a biological fluid.

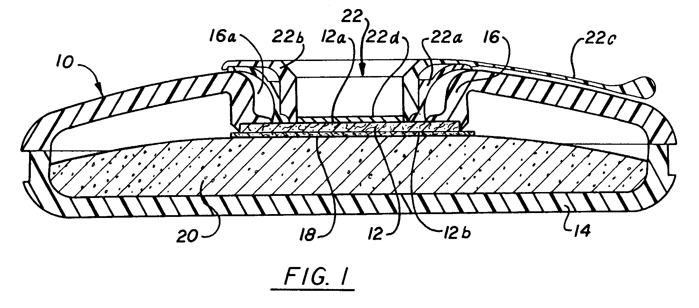

Generally the device and method employ an improved form of a solid-phase immunoassay technique for performing colorimetric or other enzyme immunoassay analysis of biological fluids. The device as depicted in Fig. 1 shows a cross-sectioned view of the various components necessary to understanding the subject improvements.

The device 10 comprises a substantially planar, generally circular, disk-shaped reaction matrix 12. The matrix 12 contains the special particles of the invention, as described herein, and is disposed within the device 10 such that the various chemical reactions and changes necessary to perform a binding assay can take place therein for visual or instrumental detection.

The matrix 12 has a sample-contacting surface 12a and a surface 12b opposed therefrom. The filter matrix 12 is a "porous" filter matrix meaning that the matrix is composed of a material into which fluids can flow and easily pass through. Appropriate materials can include glass fibers, cellulose, nylon, or other fibrous materials well know in the art. One perferred material is "Whatman GF/D" glass fiber filter paper (Whatman Reeve Angel, Inc., Clifton, New Jersey) which has a nominal thickness of 0,81mm (0.032 inch); however, thickness is not a critical factor.

The device 10 additionally includes a carrier 14 within which the matrix 12 is disposed. The carrier 14 can be made of any suitable material such as plastic, metal or other rigid or semi-rigid substance. Especially preferred as a material for the carrier 14 is a plastic commercially known as "ABS", and available from the Monsanto Company, St. Louis, Missouri. In the preferred embodiment shown, the carrier 14 completely surrounds the matrix 12 and functions as a support and holder therefore. In order to accomplish this function, the carrier 14 has a generally circular flange 16 for supporting and tightly holding the matrix 12. A fluid chamber is generally defined by sidewalls formed by an outer wall surface 16a of the flange 16 and a base wall formed by the sample contacting surface 12a of the matrix 12.

The device 10 further comprises absorbent means 20 disposed in the carrier 14, as shown, for absorbing fluids during use of the assay device. The absorbent means 20 of the device 10 can comprise one or more layers of material and is in physical contact, as shown, with the barrier material 18, when used, or with the reaction matrix 12. This especially advantageous feature enables excess fluid, during the performance of an assay using the device 10, to be easily absorbed, as necessary, after passage of such excess fluid from the reaction matrix 12 during the assay procedure. The absorbent means 20 can be virtually any moisture of fluid-retaining material, e.g., that available from James River, and designated "105 point" or "50 point", or, as is especially preferred, a combination of one or more layers of each of the foregoing.

Barrier means is provided for restricting fluid flow in the solid phase analytical devices. This aspect is particularly advantageous when used in solid phase analytical devices having a permeable reaction surface or matrix, or filter layer, and an absorbant layer for absorbing fluids used in the device to permit the flow of fluids from the reaction surface to the absorbant means or layer while preventing the back flow of fluids from the absorbant layer to the reaction matrix.

As shown in Fig. 1,the barrier means comprises a layer of barrier material 18 extending under the matrix 12 and within the carrier 14. The barrier material 18 is in contact with the surface 12b of the matrix 12, and functions, when the device is in use, to restrict fluid passing through the matrix 12, to and through the surface 12b, and into the layer 18, from re-contacting the surface 12b. Layer 18 is employed as a fluid restrictive layer and to help prevent or eliminate "background" interference in the matrix 12. However, this feature is not essential or critical to the basic functions of concepts of the matrix 12, and can be omitted from the device if desired. If omitted, the device generally will perform satisfactorily in an assay, but possibly with less sensitivity (diminished detectable response).

The layer 18 can comprise any suitable material capable of restrictive, substantially "one-way" flow of fluid or moisture. Examples of especially suitable materials for this purpose are polyethylene weave materials manufactured and sold by Ethyl Visqueen Corp., Baton Rouge, Louisiana under the designations "X-6057", 25,4$\mu$m (1.0 mil) and "X-6108", 31,75$\mu$m (1.25 mil) as well as those materials described in U.S. Patents 3,939,135 and 4,342,314. Another suitable material is "Lydair™ Grade 254" from Lydall, Inc., Manchester, Connecticut.

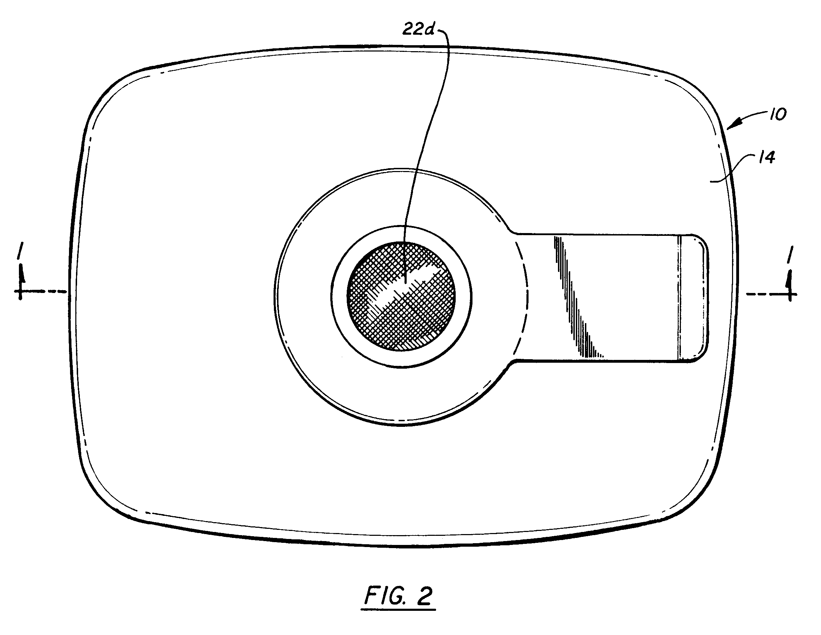

Referring now in more detail to Figs. 1, 2 and 5 of the drawings, the analytical device 10 of the invention can optionally include a filtering means 22 disposed over surface 12a of the reaction matrix 12. The filtering means 22 can be press-fitted into the carrier 14 by means of a retaining ring 22a, and preferably has a removable portion 22b having a handle portion 22c. The means 22 is further composed, for example, of a suitable porous of fibrous material 22d such as a glass or cellulose filter membrane in a plastic surround; especially preferred are "Lydair™ Grade 254" from Lydall, and "GF/F" or "GF/D" from Whatman Reeve Angel, Inc., Clifton, New Jersey, either singly or in combination.

Even though the device 10, as described above, can produce a visually-readable response, an instrumental determination can also be made of a detectable response therefrom, e.g., corresponding to the reflectance of visible light, or intensity of fluorescence or the like, produced by the matrix 12 as a result of the chemical and biological reactions and changes which occur therein when an assay is performed. Accordingly, the detectable response from the device 10 can be measured by, for example, a reflectometer.

A generalized example of how a direct diagnositc assay is performed with the instant device is as follows:

Step a) Application of a test sample containing the antigen or antibody to be determined to the reaction matrix having microparticles immobilized thereon;

Step b) Application of an enzyme-conjugated antibody or antigen to the antigen or antibody of Step a);

Step c) Washing, to remove unbound material; and

Step d) Application of an indicator substance which, in the presence of the enzyme portion of the conjugate of Step b), produces a detectable color or other response in the reaction matrix.

In another aspect, the subject invention can be employed by incubating the sample with a plurality of the spherical, solid particles comprising polytetrafluoroethylene. After the analyte becomes bound to the particles, the particles are contacted with a porous, fibrous matrix, whereby at least a portion of the particles become retained and immobilized within the matrix. The bound particles are then treated with an enzyme-conjugated antibody or antigen. The particles are washed to remove excess enzyme-conjugate and an indicator substance is added to produce a detectable color or other response. In yet another aspect, the subject spherical, solid particles comprising polytetrafluoroethylene can be employed as the solid phase components in the methods described in U.S. Patents 4,497,899 and 4,497,900.

In the subject invention, the methods and device are characterized by employing a plurality of substantially spherical, solid particles comprising polytetrafluoroethylene to bind the analyte. An example of polytetrafluoroethylene particles are Teflon™ particles, a trademark of DuPont E.I. De Nemours & Co., Wilmington, Delaware. Preferably the particles are homopolymers of polytetrafluorethylene; however, derivations of polytetrafluoroethylene can also be employed.

The polytetrafluoroethylene particles are deposited into a fiber matrix (either before or after binding the antigen, depending upon the method employed) where they are retained and immobilized. "Retained and immobilized" means that the particles, once upon the fibers of the material, are not capable of substantial movement to positions elsewhere within the material, (i.e., to other fibers), or cannot be removed completely from the material without destruction thereof. No special treatment is necessary to retain and immobilize the particles on the fiber matrix, as they have been found to readily adhere to the fibers upon exposure thereto.

The particles have an average individual diameter of from about 0.1 to about 10 $\mu$m more preferably from about 2 to about 5 $\mu$m in diameter and an average density of about 2.14 g/cm$^3$. It is to be recognized that the particles can be present as aggregates which can exceed 200 $\mu$m in diameter. The particles are substantially uncoated and in fact it has been found that this is the preferred method for performing the assay for Chlamydia and gonorrhoeae antigens. However, further embodiments can include coating the particles with antibody or other substances to assist in the binding of antigen.

The polytetrafluoroethylene microparticles are extremely stable under a variety of conditions and therefore can be employed with a wide range of samples containing the Chlamydia or gonorrhoeae antigens. For example, the microparticles are resistant to strong acids and bases as well as most organic solvents. The microparticles are also stable from -240° to 260°C and because they are hydrophobic they absorb minimal amounts of water.

In the performance of an immunoassay for Chlamydia and gonorrhoeae it has been discovered that the polytetrafluoroethylene microparticles provide superior results as compared to other potential polymeric particles. This is demonstrated in the following examples:

Example I

Various microparticles were compared for their ability to bind antigen in an enzyme immunoassay specific for Chlamydia.

In the method, Chlamydia antigens were absorbed by microparticle suspensions. The bound analyte was separated from unbound analyte by centrifugation and the absorbed analyte detected using rabbit anti-Chlamydia and goat anti-rabbit globulin conjugated to horseradish peroxide to detect immune complexes. The indicator employed were ortho phenylene diamine. Following color development the microparticles were removed by centrifugation and the supernatants transferred to cuvettes for measuring relative absorbance at 492 nm with a spectrophotometer.

In this experiment, the capacity of each type of microparticle to absorb analyte in a suspension containing Chlamydia was measured. Non-specific binding was also evaluated by testing in parallel each type of microparticle in diluent solutions containing no analyte (blank)

The results obtained for seven types of microparticles were as follows:

| Microparticle | Diameter ($\mu$m) | % Solids | Absorbency at 492 nm Chlamydia 5 x 10$^6$/ml | Blank |
|---|---|---|---|---|
| polystyrene (PS) | .55 | 0.2 | .144 | .064 |
| carboxylated PS | .47 | 0.2 | .087 | .097 |
| polymyxin B-PS | .47 | 0.2 | .127 | .022 |
| wheat germ agg. PS | .47 | 0.2 | .066 | .027 |
| glycine PS | .47 | 0.2 | .055 | .032 |
| cationic PS | .22 | 0.2 | .021 | .012 |
| polytetrafluoro-ethylene | 2-5 | 0.1 | 1.555 | .021 |

The results show that the polytetrafluoroethylene microparticle has a superior capacity to absorb analyte when exposed to the microorganism. Also the polytetrafluoroethylene showed low non-specific binding.

Example II

The sensitivity of the polytetrafluoroethylene particles was further demonstrated by measuring the absorbancy at 492 nm when exposed to decreasing levels of microorganism. A similar centrifugal enzyme immunoassay technique was employed as in Example I. The absorbancy measured at varying concentrations of Chlamydia is shown below:

| Chlamydia (organism/ml) | Absorbancy at 492nm |
|---|---|
| $5 \times 10^6$ | 1.555 |
| $5 \times 10^5$ | 0.609 |
| $5 \times 10^4$ | 0.073 |
| none | 0.021 |

The results indicate excellent sensitivity to as low as 50,000 organisms per millimeter with over a three-fold difference between the blank and the sample at this level.

Example III

The sensitivity of the polytetrafluoroethylene particles was further evaluated in the instant solid-phase device (TESTPACK™) by immobilizing microparticles in the glass fibrous matrix to form the reaction matrix. The reaction matrix was then treated with Chlamydia microorganisms at various levels of concentration and then visual colormetric change and reflectance was measured after treatment with an indicator substance.

The results were as follows:

| Sample Concentration Chlamydia/ml | Signal | |
|---|---|---|
| | Visual | % Reflectance |
| $5 \times 10^7$ | 4 | 15.1 |
| $5 \times 10^6$ | 4 | 22.1 |
| $5 \times 10^5$ | 3 | 31.1 |
| $5 \times 10^4$ | 2 | 45.6 |
| $5 \times 10^3$ | 1 | 49.4 |
| $5 \times 10^2$ | + | 53.3 |
| Blank | – | 58.4 |

The signal was measured visually on a scale of 1 to 4 for color intensity for positive results with 4 being a blue-black color and 1 being a light blue color. The signal was also quantified by a reflectometer which measured percent reflectance. The perecent reflectance corresponded to the color intensity with the lowest reflectance indicating a very dark color.

The results indicate that a positve result could be detected for as low as $5 \times 10^2$ microorganisms per milliliter. Visually, at the $5 \times 10^2$ microorganism level a slight color was perceptable to the point a positive reaction was indicated although not within the color scale; however, the instrumentation signal showed a definite difference between the blank and the $5 \times 10^2$ microorganisms per milliliter level. These results show an excellent sensitivity of the subject device and method to low concentrations of Chlamydia.

While all the above examples have employed Chlamydia, the device and method is equally applicable to the identification of gonorrhoeae antigens.

The subject invention can also provide "on-board" control areas to simultaneously display detectable responses corresponding to a positive control (which will display a detectable response indicative of a valid assay result, regardless of the presence or absence of an analyte of interest in a test sample), and a negative control (which will display a detectable response change only if the assay results are invalid). In this aspect of the invention, the same volume of a test sample and assay reagents are simultaneously placed in contact with the procedural controls and test areas, thereby avoiding the necessity of separate control tests as generally practiced in the art.

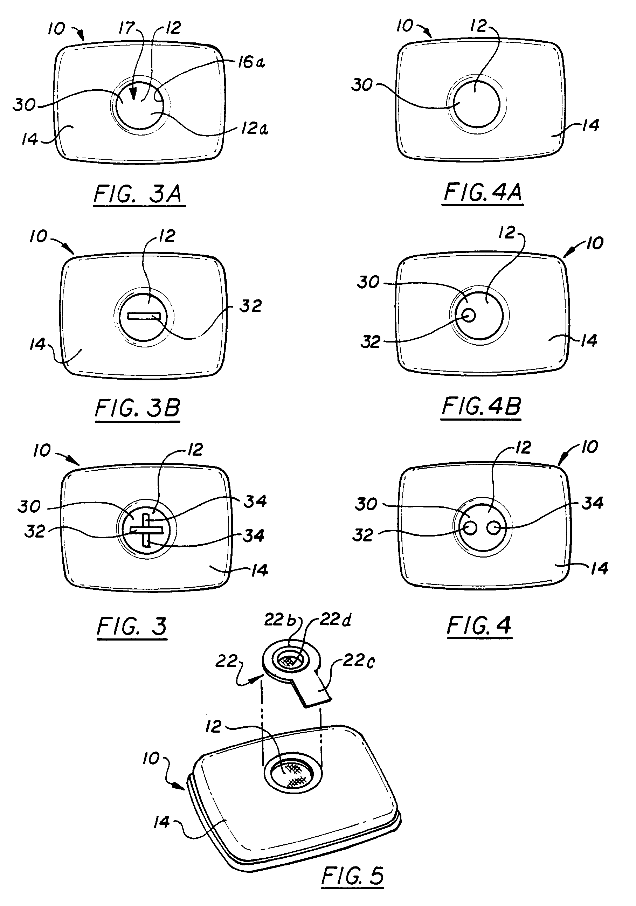

Referring to Figures 3A-C and 4A-C on-board negative and positive control areas 30 and 32, respectively, can be provided on the reaction surface or matrix 12 of the analytical device 10. The analyte binding area 34 indicates a positive test result to the user. Generally the control areas 32 and 34 are configured to provide a readily recognizable result to the user as shown in Figure 3C.

The negative control area 30 is formed by maintaining the area 30 free of substances which will retain the enzyme label or other signal response material during the assay. The positive control area 32 is formed by providing a substance capable of binding the enzyme label or other signal response material regardless

EP 0 264 036 B1

of the presence of the antigen of interest. The analyte binding area 34 is formed by coating the microparticles of polytetrafluoroethylene at this position to bind the antigen of interest. In other embodiments the device can contain only the analyte binding area 34 and other configurations of areas 30, 32, and 34 can be employed.

**Claims**

1. A solid-phase assay device (10) useful in a binding assay for determining the presence of an analyte in a sample, the device comprising a substantially planar layer (12) of a material having a porous matrix of fibers and a plurality of substantially spherical, solid particles having an average diameter of from 0.1 to 10μm the particles being retained and immobilized within said matrix upon the fibers, the substantially planar layer (12) having a first, sample-contacting surface (12a) and a second surface (12b) opposed to the first surface such that, when the device is in use in the performance of the assay, at least a portion of the sample contacting the first surface (12a) passes through the substantially planar layer to the second surface (12b), characterized in that said solid particles consist essentially of polytetrafluoroethylene capable of directly binding Chlamydia trachomatis or Neisseria gonorrhoeae.

2. The assay device of Claim 1, wherein the device additionally comprises filtering means (22) disposed in relationship to the first surface (12a) of the substantially planar layer (12), such that sample fluid passes through said filtering means (22) prior to contacting the first surface (12a).

3. The assay device of Claim 1, wherein the device additionally comprises absorbent means (20) disposed in relationship to the lower surface (12b) of the substantially planar layer (12), such that at least a portion of the fluid passing through the substantially planar layer (12) is absorbed by the absorbent means (20).

4. The assay device of Claim 1, wherein said substantially planar layer (12) further comprises a reaction surface having a negative control area (30), a positive control area (32) and an analyte binding area (34) configured so that the positive control area (32) interacts with the analyte binding area (34) to form a first representational symbol having a first meaning upon the occurrence of a positive test result in the use of the device and to form a second representational symbol different from that of the first representational symbol upon the occurrence of a negative test result in the use of the device.

5. The assay device of Claim 4, wherein the positive control area (32) is formed on the reaction surface in the shape of a rectangular bar.

6. The assay device of Claim 5, wherein the analyte binding area (34) is formed on the reaction surface in the shape of two rectangular bars on opposite sides of and oriented perpendicularly to the positive control area (32), whereby the analyte binding area (34) taken together with the positive control area (32) form the shape of a "+" sign and where the positive control area (32) acts alone to form a "-" symbol upon the occurrence of a negative test result.

7. The assay device of Claim 1 wherein said spherical, solid particles are a homopolymer of polytetrafluoroethylene.

8. A binding assay method employing a solid phase capable of binding Chlamydia trachomatis or Neisseria gonorrhoeae antigens in a fluid sample, comprising contacting the fluid sample with a solid phase consisting essentially of polytetrafluoroethylene particles, wherein Chlamydia trachomatis or Neisseria gonorrhoeae antigens become bound directly to the solid phase.

9. The method of Claim 8, wherein said polytetrafluoroethylene particles have an average diameter of from 0.1 to 10μm.

10. The method of Claim 8, wherein said polytetrafluoroethylene particles are homopolymers of polytetrafluoroethylene.

11. The method of Claim 8, wherein, following said contacting, said particles are deposited into a fibrous matrix, wherein they are retained.

7

**12.** A method according to Claim 11, comprising the steps of:

a) incubating with a sample of the fluid a plurality of substantially spherical, solid particles comprising polytetrafluoroethylene having an average diameter of from 0.1 to 10μm, whereby the antigen becomes bound to the particles forming an antigen complex on the particles; and

b) contacting a porous, fibrous matrix with the incubated particles, whereby at least a portion of the particles become retained and immobilized within the matrix upon at least a portion of the fibers.

c) detecting the presence of antigen bound to the particles.

**13.** The method of Claim 8, wherein, prior to said contacting, said particles are deposited into a fibrous matrix, wherein they are retained.

**14.** A method according to Claim 13 comprising the steps of:

a) contacting a porous matrix of fibers and a plurality of substantially spherical, solid particles comprising polytetrafluoroethylene having an average diameter of from 0.1 to 10μm, the particles being retained and immobilized within said matrix upon the fibers, whereby antigen in the sample becomes bound to the particles forming an antigen complex on the particles;

b) contacting the antigen complex on the particles with a second substance capable of reaction with the antigen, which is labeled with a substance capable of producing a detectable response in the presence of the antigen and an indicator substance, whereby the labeled substance becomes bound to the complex on the particles;

c) removing umbound labeled substance from the material;

d) contacting the complex with the indicator substance; and

e) detecting the response produced as a function of the presence or amount of the antigen in the sample.

## Patentansprüche

**1.** Festphasen-Assay-Vorrichtung (10), die geeignet ist für Bindungs-Assays zur Bestimmung des Vorhandenseins eines Analyten in einer Probe, wobei die Vorrichtung umfaßt eine im wesentlichen ebene Schicht (12) aus einem Material, das eine poröse Matrix aus Fasern und eine Vielzahl von im wesentlichen sphärischen festen Teilchen mit einem mittleren Durchmesser von 0,1 bis 10 μm aufweist, wobei die Teilchen in der Matrix auf den Fasern festgehalten und immobilisiert sind, die im wesentlichen ebene Schicht (12) eine erste, mit der Probe in Kontakt kommende Oberfläche (12a) und eine zweite, der ersten Oberfläche gegenüberliegende Oberfläche (12b) aufweist, so daß, wenn die Vorrichtung bei der Durchführung des Assays angewandt wird, mindestens ein Teil der Probe, die mit der ersten Oberfläche (12a) in Berührung kommt, durch die im wesentlichen ebene Schicht zu der zweiten Oberfläche (12b) hindurchgeht, dadurch gekennzeichnet, daß die festen Teilchen im wesentlichen aus Tetrafluorethylen bestehen, das in der Lage ist, Chlamydia trachomatis oder Neisseria gonorrhoeae direkt zu binden.

**2.** Assay-Vorrichtung nach Anspruch 1, wobei die Vorrichtung zusätzlich ein Filtermittel (22) umfaßt, das in Beziehung zu der ersten Oberfläche (12a) der im wesentlichen ebenen Schicht so angeordnet ist, daß die Probenflüssigkeit durch das Filtermittel (22) hindurchgeht, bevor sie mit der ersten Oberfläche (12a) in Kontakt kommt.

**3.** Assay-Vorrichtung nach Anspruch 1, wobei die Vorrichtung zusätzlich ein Absorptionsmittel (20) umfaßt, das in Beziehung zu der unteren Oberfläche (12b) der im wesentlichen ebenen Schicht (12) so angeordnet ist, daß mindestens ein Teil der Flüssigkeit, die durch die im wesentlichen ebene Schicht (12) hindurch geht, von dem Absorptionsmittel absorbiert wird.

**4.** Assay-Vorrichtung nach Anspruch 1, wobei die im wesentlichen ebene Schicht (12) ferner eine Reaktions-Oberfläche umfaßt mit einem negativen Kontroll-Bereich (30), einem positiven Kontroll-Bereich (32) und einem Analyt-Bindungsbereich (34) umfaßt, um ein erstes repräsentatives Symbol für einen ersten Anschein beim Auftreten eines positiven Test-Ergebnisses bei der Anwendung der Vorrichtung zu ergeben. und um ein zweites repräsentatives Symbol zu ergeben, das von dem ersten repräsentativen Symbol verschieden ist, beim Auftreten eines negativen Test-Ergebnisses bei der Anwendung der Vorrichtung.

**5.** Assay-Vorrichtung nach Anspruch 4, wobei der positive Kontroll-Bereich (32) auf der Reaktions-Oberfläche in Form eines rechteckigen Balkens gebildet wird.

**6.** Assay-Vorrichtung nach Anspruch 5, wobei der Analyt-Bindungsbereich (34) auf der Reaktions-Oberfläche in Form von zwei rechteckigen Balken auf gegenüberliegenden Seiten des positiven Kontroll-Bereichs (32) gebildet und senkrecht zu diesem orientiert ist, wobei der Analyt-Bindungs-Bereich (34) zusammen mit dem positiven Kontroll-Bereich (32) ein "+"-Zeichen bildet und wobei der positive Kontroll-Bereich (32) allein so wirkt, daß er ein "-"-Zeichen bildet beim Auftreten eines negativen Test-Ergebnisses.

**7.** Assay-Vorrichtung nach Anspruch 1, wobei die sphärischen festen Teilchen ein Homopolymer aus Polytetrafluorethylen sind.

**8.** Bindungs-Assay-Verfahren unter Verwendung einer festen Phase, die in der Lage ist, Chlamydia trachomatis oder Neisseria gonorrhoeae Antigene in einer flüssigen Probe zu binden, umfassend das Zusammenbringen der flüssigen Probe mit einer festen Phase, bestehend im wesentlichen aus Polytetrafluorethylen-Teilchen, wobei Chlamydia trachomatis oder Neisseria gonorrhoeae Antigene direkt an die feste Phase gebunden werden.

**9.** Verfahren nach Anspruch 8, wobei die Polytetrafluorethylen-Teilchen einen mittleren Durchmesser von 0,1 bis 10 $\mu$m besitzen.

**10.** Verfahren nach Anspruch 8, wobei die Polytetrafluorethylen-Teilchen Homopolymere aus Polytetrafluorethylen sind.

**11.** Verfahren nach Anspruch 8, wobei nach dem Zusammenbringen die Teilchen in einer faserigen Matrix abgeschieden werden, wo sie festgehalten werden.

**12.** Verfahren nach Anspruch 11, umfassend die folgenden Stufen:
a) Inkubieren einer Vielzahl von im wesentlichen sphärischen festen Teilchen, umfassend Polytetrafluorethylen mit einem mittleren Durchmesser von 0,1 bis 10 $\mu$m, mit einer Probe der Flüssigkeit, wodurch das Antigen an die Teilchen gebunden wird unter Bildung eines Antigen-Komplexes auf den Teilchen,
b) Zusammenbringen einer porösen faserförmigen Matrix mit den inkubierten Teilchen, wobei zumindest ein Teil der Teilchen in der Matrix auf mindestens einem Teil der Fasern festgehalten und immobilisiert wird,
c) Nachweis des Vorhandenseins von Antigen, das an die Teilchen gebunden ist.

**13.** Verfahren nach Anspruch 8, wobei vor dem Zusammenbringen die Teilchen in einer faserigen Matrix abgeschieden werden, wo sie festgehalten werden.

**14.** Verfahren nach Anspruch 13, umfassend die folgenden Stufen:
a) Zusammenbringen einer porösen Matrix aus Fasern mit einer Vielzahl von im wesentlichen sphärischen festen Teilchen, umfassend Polytetrafluorethylen mit einem mittleren Durchmesser von 0,1 bis 10 $\mu$m, wobei die Teilchen innerhalb der Matrix auf den Fasern festgehalten und immobilisiert werden, wodurch das Antigen in der Probe an die Teilchen gebunden wird unter Bildung eines Antigen-Komplexes auf den Teilchen,
b) Zusammenbringen des Antigen-Komplexes mit einer zweiten Substanz, die mit dem Antigen reagieren kann und die markiert ist mit einer Substanz, die in der Lage ist, eine nachweisbare Reaktion in Gegenwart von dem Antigen und einer Indikatorsubstanz hervorzurufen, wodurch die markierte Substanz an den Komplex auf den Teilchen gebunden wird,
c) Entfernen von nicht gebundener markierter Substanz von dem Material,
d) Zusammenbringen des Komplexes mit der Indikatorsubstanz und
e) Nachweis der Reaktion, die auftritt als Funktion des Vorhandenseins oder der Menge an Antigen in der Probe.

# EP 0 264 036 B1

**Revendications**

1. Dispositif de dosage en phase solide (10) utile dans une analyse par fixation pour déterminer la présence d'un analyte dans un échantillon, le dispositif comprenant une couche pratiquement plane (12) d'un matériau ayant une matrice poreuse de fibres et une pluralité de particules solides pratiquement sphériques ayant un diamètre moyen allant de 0,1 à 10 $\mu$m, les particules étant retenues et immobilisées dans ladite matrice sur les fibres, la couche pratiquement plane (12) ayant une première surface en contact avec l'échantillon (12a) et une seconde surface (12b) opposée à la première surface de telle façon que, quand le dispositif est en fonction pour réaliser le dosage, au moins une partie de l'échantillon entrant en contact avec la première surface (12a) traverse la couche pratiquement plane pour aller sur la seconde surface (12b), caractérisé en ce que lesdites particules solides sont constituées essentiellement par du polytétrafluoroéthylène capable de fixer directement <u>Chlamydia trachomatis</u> ou <u>Neisseria gonorrhoeae.</u>

2. Dispositif de dosage de la revendication 1, dispositif comprenant en outre des moyens de filtration (22) disposés en relation avec la première surface (12a) de la couche pratiquement plane (12), de telle façon que le fluide de l'échantillon traverse ledit moyen de filtration (22) avant de rentrer en contact avec la première surface (12a).

3. Dispositif de dosage de la revendication 1, dispositif comprenant en outre un moyen d'absorption (20) disposé en relation avec la surface inférieure (12b) de la couche pratiquement plane (12) de façon à ce qu'au moins une fraction du fluide traversant la couche 12 pratiquement plane soit absorbée par le moyen d'absorption (20).

4. Dispositif de dosage de la revendication 1, dans lequel ladite couche pratiquement plane (12) comprend en outre une surface réactionnelle ayant une zone témoin négative (30), une zone témoin positive (32) et une zone de fixation de l'analyte (34) configurée de façon à ce que la zone témoin positive (32) interagisse avec la zone de fixation de l'analyte (34) pour former un premier symbole représentationnel ayant une première signification en cas de résultat d'essai positif lorsqu'on utilise le dispositif, et pour former un second symbole représentationnel différent du premier symbole représentationnel en cas de résultat d'essai négatif lorsqu'on utilise le dispositif.

5. Dispositif de dosage de la revendication 4, dans lequel la zone témoin positive (32) a sur la surface réactionnelle la forme d'une barre rectangulaire.

6. Dispositif de dosage de la revendication 5, dans lequel la zone de fixation de l'analyte (34) a sur la surface réactionnelle la forme de deux barres rectangulaires situées de part et d'autre de la zone témoin positive (32) et orientées perpendiculairement à cette zone témoin positive, la zone de fixation de l'analyte (34) constituant avec la zone témoin positive (32) un signe " + " et la zone témoin positive (32), quand elle réagit seule, formant un symbole "-", en cas de résultat de test négatif.

7. Dispositif de dosage de la revendication 1 dans lequel lesdites particules solides sphériques sont un homopolymère de polytétrafluoroéthylène.

8. Méthode de dosage par fixation utilisant une phase solide capable de fixer des antigènes <u>Chlamydia trachomatis</u> ou <u>Neisseria gonorrhoeae</u> dans un échantillon fluide, comprenant la mise en contact de l'échantillon fluide avec une phase solide formée essentiellement de particules de polytétrafluoroéthylène, dans laquelle les antigènes <u>Chlamydia trachomatis</u> ou <u>Neisseria gonorrhoeae</u> se lient directement à la phase solide.

9. Méthode de la revendication 8, dans laquelle les particules de polytétrafluoroéthylène ont une diamètre moyen allant de 0,1 à 10 $\mu$m.

10. Méthode de la revendication 8 dans laquelle les particules de polytétrafluoroéthylène sont des homopolymères du polytétrafluoroéthylène.

11. Méthode de la revendication 8 dans laquelle après ledit contact, lesdites particules sont déposées sur une matrice fibreuse, sur laquelle elles sont retenues.

10

**12.** Méthode selon la revendication 11, comprenant les étapes de :

a) incubation, avec un échantillon de fluide, d'une pluralité de particules solides pratiquement sphériques, comprenant du polytétrafluoroéthylène ayant un diamètre moyen de 0,1 à 10 $\mu$m, incubation au cours de laquelle l'antigène se lie aux particules pour former un complexe d'antigène sur les particules ; et

b) mise en contact d'une matrice fibreuse poreuse avec les particules incubées, contact au cours duquel au moins une partie des particules est retenue et immobilisée dans la matrice sur au moins une fraction des fibres.

c) détection de la présence de l'antigène lié aux particules.

**13.** Méthode de la revendication 8, dans laquelle, avant ledit contact, lesdites particules sont déposées sur une matrice fibreuse dans laquelle elles sont retenues.

**14.** Méthode selon la revendication 13 comprenant les étapes de :

a) mise en contact d'une matrice poreuse de fibres et d'une pluralité de particules solides pratiquement sphériques comprenant du polytétrafluoroéthylène ayant un diamètre moyen de 0,1 à 10 $\mu$m, les particules étant retenues et immobilisées dans ladite matrice sur les fibres, contact au cours duquel l'antigène dans l'échantillon se lie aux particules pour former un complexe d'antigène sur les particules;

b) mise en contact du complexe d'antigène sur les particules avec une seconde substance capable de réagir avec l'antigène, marquée par une substance capable de produire une réponse détectable en présence de l'antigène et d'une substance indicatrice, contact au cours duquel la substance marquée se lie au complexe sur les particules ;

c) élimination de la substance marquée non liée du matériau ;

d) mise en contact du complexe avec la substance indicatrice ; et

e) détection de la réponse produite en fonction de la présence ou de la quantité de l'antigène dans l'échantillon.

*FIG. I*

_FIG. 2_

FIG. 3A

FIG.4A

FIG. 3B

FIG.4B

FIG. 3

FIG. 4

FIG. 5